(19) Europäisches Patentamt / European Patent Office / Office européen des brevets

(11) **EP 2 332 462 B1**

(12) **FASCICULE DE BREVET EUROPEEN**

(45) Date de publication et mention
de la délivrance du brevet:
**06.04.2016 Bulletin 2016/14**

(21) Numéro de dépôt: **10182352.4**

(22) Date de dépôt: **29.09.2010**

(51) Int Cl.:
*A61N 1/39* *(2006.01)*       *A61N 1/05* *(2006.01)*
*A61B 5/04* *(2006.01)*       *A61B 5/0452* *(2006.01)*
*A61B 5/0464* *(2006.01)*

(54) **Défibrillateur/cardioverteur implantable simple chambre avec détection de l'activité auriculaire par la sonde monocorps**

Einfacher implantierbarer Einkammer-Defibrillator/Kardioverter mit Erfassung der aurikularen Aktivität über Monokörper-Sonde

Single-chamber implantable cardioverter/defibrillator with detection of atrial activity via the single-body probe

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**

(30) Priorité: **11.12.2009 FR 0958898**

(43) Date de publication de la demande:
**15.06.2011 Bulletin 2011/24**

(73) Titulaire: **Sorin CRM SAS**
**92143 Clamart Cedex (FR)**

(72) Inventeurs:
• **Milpied, Paola**
**75014 PARIS (FR)**

• **Henry, Christine**
**75014 PARIS (FR)**

(74) Mandataire: **Dupuis-Latour, Dominique et al**
**Bardehle Pagenberg**
**10, boulevard Haussmann**
**75009 Paris (FR)**

(56) Documents cités:
**EP-A1- 0 278 864      EP-A1- 1 118 349**
**EP-A1- 2 105 843      EP-A2- 0 801 960**
**EP-A2- 0 879 621      US-A1- 2005 288 600**

**Description**

L'invention concerne les "dispositifs médicaux implantables actifs" tels que définis par la directive 90/385/CEE du 20 juin 1990 du Conseil des communautés européennes.

Elle concerne plus particulièrement la famille des appareils capables de mettre fin à une tachyarythmie en délivrant au coeur un choc sous forme d'impulsions électriques de haute énergie, dépassant notablement le niveau nécessaire à une simple stimulation.

Ces dispositifs sont généralement appelés cardioverteurs/défibrillateurs implantables ou ICDs.

Ils sont constitués de deux ensembles, à savoir un générateur d'impulsions et une sonde ou un système de sondes.

Le générateur est chargé de surveiller l'activité cardiaque et de générer des impulsions de haute énergie quand le coeur présente une arythmie ventriculaire susceptible d'être traitée par l'application d'un choc de défibrillation (ce mot incluant, dans la présente description, aussi bien la cardioversion que la défibrillation, si des significations différentes sont donnés à ces deux termes).

Au générateur sont connectées une ou plusieurs sondes munies d'électrodes dont le rôle est de distribuer au coeur cette énergie de façon appropriée. Le EP 0 773 039 A1 (ELA Médical) décrit un tel ensemble générateur/sonde, avec des moyens permettant de choisir la configuration optimale d'application du choc de défibrillation.

L'invention concerne le cas particulier où le générateur est relié à une sonde dite "monocorps", qui est une sonde unique portant les différentes électrodes permettant à la fois de surveiller l'activité cardiaque et de délivrer le choc de cardioversion/défibrillation.

Le problème de l'invention est le recueil, avec une telle sonde monocorps, d'un signal représentatif de l'activité auriculaire.

En effet, un choc de défibrillation ne doit être délivré que dans le cas d'une véritable tachycardie ventriculaire (TV), et non d'une tachycardie supra-ventriculaire (TSV) : en effet, dans ce dernier cas la tachycardie est d'origine auriculaire, et le choc qui serait délivré au ventricule serait sans effet puisque l'électrode de défibrillation ne se trouve pas dans cette région. De plus, l'application d'un choc de défibrillation chez un patient conscient est extrêmement douloureuse et angoissante, les énergies appliquées étant en effet très au-delà du seuil de douleur, sans compter que l'application d'un choc de défibrillation n'est pas dénuée d'effets secondaires sur le rythme cardiaque (risque d'apparition de troubles secondaires), sur l'intégrité fonctionnelle du myocarde, et de façon générale sur l'équilibre physiologique du patient.

Il est donc important de ne délivrer de tels chocs que de façon appropriée et seulement si une autre thérapie moins douloureuse, telle qu'une stimulation appropriée de l'oreillette, n'est pas envisageable.

L'analyse de l'activité auriculaire, qui implique en particulier la reconnaissance des ondes P, est donc un point fondamental dans ce domaine.

A cet égard, lorsque le défibrillateur est un défibrillateur double chambre, il comprend des moyens propres de détection du rythme cardiaque auriculaire, à partir duquel il est aisé de détecter une situation de fibrillation auriculaire telle qu'une TSV et donc inhiber la délivrance d'une thérapie de choc au ventricule.

En revanche, lorsque le défibrillateur est un défibrillateur simple chambre, il ne comporte pas de moyen de détecter l'activité extrêmement rapide des oreillettes, de sorte que si la fréquence ventriculaire est suffisamment rapide, un choc ventriculaire inapproprié sera inévitable.

Or la pose d'un défibrillateur simple chambre est suffisante dans nombre de cas, notamment chez les patients pour lesquels un défibrillateur est indiqué mais dont le noeud sinusal ne présente aucune dysfonction. On notera à cet égard que l'implantation d'un défibrillateur double chambre, s'il permet certes d'améliorer les performances en ce qui concerne la classification des tachyarythmies (discrimination entre FV et TSV), multiplie par deux le risque de complications liées aux sondes.

Le but de la présente invention est de proposer un moyen de recueillir et extraire l'activité auriculaire avec un défibrillateur simple chambre muni d'une unique sonde ventriculaire, notamment une sonde monocorps standard.

L'état de la technique contient de nombreuses propositions de dispositifs médicaux et notamment de sondes destinées à recueillir un signal de détection auriculaire avec une sonde de défibrillation.

Les US 4 643 201 A (Medtronic), US 5 628 779 A (Pacesetter Inc.) et US 6 321 122 A (Cardiac Pacemakers Inc.) décrivent diverses formes de sondes comportant une ramification ou un coude munis d'une électrode spécifique, localisée au niveau de l'oreillette ou au voisinage de celle-ci une fois la sonde implantée.

Le EP 0 801 960 A2 décrit un autre type de sonde très particulière, avec une partie flottante dans l'oreillette, une paire d'électrodes bipolaires, une partie distale dans le ventricule et une électrode distale.

Dans tous les cas, il s'agit de sondes dédiées, relativement complexes et spécifiques, dont l'utilisation ne peut pas être généralisée. D'autre part, l'électrode située au niveau de l'oreillette reste une électrode flottante, délivrant un signal auriculaire bruité, de qualité médiocre, rendant difficile l'analyse du rythme auriculaire.

Une autre proposition est celle du EP 1 118 349 A1 (ELA Medical), qui propose une sonde de type monocorps, non ramifiée et non coudée, pourvue dans sa région proximale de deux électrodes auriculaires, ainsi que de deux électrodes ventriculaires, et une électrode supraventriculaire pour la délivrance du choc de défibrillation. Le signal auriculaire est recueilli entre, d'une part, l'électrode supraventriculaire et l'électrode auriculaire qui lui est reliée et, d'autre part, l'autre électrode auriculaire. Toutefois, même si la qualité du signal peut être améliorée, cette sonde reste un modèle non conventionnel, qui ne peut donc être mis en oeuvre de façon simple et à grande échelle.

D'autres techniques ont été proposées pour recueillir l'activité auriculaire avec des sondes monocorps standard, c'est-à-dire des sondes comprenant une (ou deux) électrodes distales de détection monopolaire (ou bipolaire, respectivement) d'un signal ventriculaire, un bobinage ou "coil" ventriculaire formant électrode de défibrillation, et un bobinage ou "coil" positionné principalement dans la veine cave supérieure (SVC, *Superior Vena Cava*), au voisinage de l'oreillette.

La difficulté dans ce cas est que le coil SVC n'est pas véritablement une électrode auriculaire et il ne permet pas le recueil convenable d'un signal cardiaque auriculaire, d'autant plus que cette électrode est flottante et délivre de ce fait un signal fortement bruité.

En particulier, le signal auriculaire recueilli sur le coil SVC (ou plus précisément, entre le coil SVC et le boîtier du générateur, en détection monopolaire) est fortement parasité par le signal ventriculaire, qui est lui aussi présent et est souvent plus important en amplitude que le signal auriculaire.

Pour discriminer ces deux composantes auriculaire et ventriculaire et extraire le signal auriculaire de plus faible amplitude, des techniques ont été proposées notamment par les US 5 776 072 A et US 5 885 221 A (Cardiac Pacemakers Inc.).

Le premier de ces documents enseigne, après détection des signaux et fenêtrage appropriés, d'estimer une fonction de transfert du signal de la voie ventriculaire par rapport à la voie combinée (ventriculaire + auriculaire), cette fonction de transfert reflétant la contribution de l'onde R aux signaux de la voie combinée. L'application de la fonction de transfert estimée aux signaux de la voie combinée est ensuite soustraite pour obtenir un signal résiduel constituant une approximation de l'onde P.

Le second document prévoit, après estimation de la fonction de transfert, d'effectuer un calcul de convolution entre le signal combiné et la fonction de transfert, pour obtenir la contribution du signal ventriculaire. Cette contribution sera ensuite déduite du signal combiné pour délivrer une estimation du signal auriculaire.

On notera que dans ces deux propositions, la discrimination des composantes auriculaire et ventriculaire est opérée par identification et soustraction du signal de l'onde R par rapport à un signal combiné mesuré par détection bipolaire entre le coil RV et le coil SVC. Les calculs exécutés sont relativement complexes, et les résultats de ces méthodes n'ont jamais été publiés, de sorte qu'il n'est pas possible de savoir si, concrètement, elles donnent une estimation satisfaisante de l'onde P, c'est-à-dire si elles sont efficaces ou non sur le plan clinique.

L'invention a pour but de pallier les difficultés exposées ci-dessus, en proposant une nouvelle technique d'analyse des signaux délivrés par une sonde de défibrillation monocorps de type conventionnel utilisée avec un défibrillateur simple chambre.

L'un des buts de l'invention est en particulier de proposer une technique qui ne nécessite pas de concevoir une nouvelle sonde ventriculaire, et qui puisse être utilisée avec les sondes préexistantes, à savoir les sondes monocorps standard "double coil" (coil RV et coil SVC) dépourvues au niveau de l'oreillette de toute électrode spécifique qui permettrait de recueillir un signal très local (comme cela a été décrit dans diverses propositions de sondes spécifiques de l'art antérieur).

Un autre but de la présente invention est de permettre ainsi à un défibrillateur automatique implantable "simple chambre" d'effectuer une détection de type "double chambre" à partir des signaux mesurés avec une sonde telle qu'exposée ci-dessus, avec des performances de détection du signal auriculaire qui soient suffisantes pour permettre une classification des tachyarythmies.

L'utilisation de dispositifs médicaux standard, simples, procure de nombreux avantages : coût réduit au minimum, procédure d'implantation rapide et simple, minimisation des risques liés aux sondes. Essentiellement, l'invention propose de recueillir deux signaux unipolaires, à savoir les signaux coil RV/boîtier et coil SVC/boîtier, et d'effectuer sur ces signaux un traitement de type "analyse en composantes indépendantes" (analyse ACI ou ICA), permettant discriminer les deux signaux statistiquement indépendants que sont le signal auriculaire et le signal ventriculaire.

L'intégration d'un traitement ACI à un défibrillateur implantable a été proposée par le US 2005/0288600 A1 mais à d'autres fins, pour assurer notamment une séparation entre une source de signal cardiaque et une source de bruit parasite.

L'invention propose un défibrillateur/cardioverteur du type général divulgué par le US 2005/0288600 A1 précité, correspondant au préambule de la revendication 1. Les éléments propres à l'invention sont énoncés dans la partie caractérisante.

Les sous-revendications visent des formes de réalisation particulières, avantageuses.

On va maintenant décrire un exemple de mise en oeuvre du dispositif de l'invention, en référence aux dessins annexés où les mêmes références désignent d'une figure à l'autre des éléments identiques ou fonctionnellement semblables.

La Figure 1 est une vue schématique d'un défibrillateur simple chambre implanté chez un patient, avec sa sonde monocorps de type "double coil".
La Figure 2 donne des exemples de chronogrammes représentatifs des signaux recueillis au moyen de cette sonde, et des signaux traités par mise en oeuvre de l'invention.
La Figure 3 est un schéma par blocs explicitant le mode de mise en oeuvre de l'invention.

On va maintenant décrire un exemple de réalisation du dispositif de l'invention.

En ce qui concerne ses aspects logiciels, l'invention peut être mise en oeuvre par une programmation appropriée du logiciel de commande d'un stimulateur connu, par exemple de type stimulateur cardiaque, resynchroniseur et/ou défibrillateur, comprenant des moyens d'acquisition d'un signal fourni par des sondes endocavitaires et/ou un ou plusieurs capteurs implantés.

L'invention peut notamment être appliquée aux dispositifs implantables tels que ceux des familles *Paradym* et *Ovatio* produits et commercialisés par ELA Médical (Sorin Group).

Il s'agit de dispositifs à microprocesseur programmable comportant des circuits pour recevoir, mettre en forme et traiter des signaux électriques recueillis par des électrodes implantées, et délivrer des impulsions à ces électrodes. Il est possible d'y transmettre par télémétrie des logiciels qui seront conservés en mémoire et exécutés pour mettre en oeuvre les fonctions de l'invention qui seront décrites ci-dessous. L'adaptation de ces appareils à la mise en oeuvre des fonctions de l'invention est à la portée de l'homme du métier, et elle ne sera pas décrite en détail.

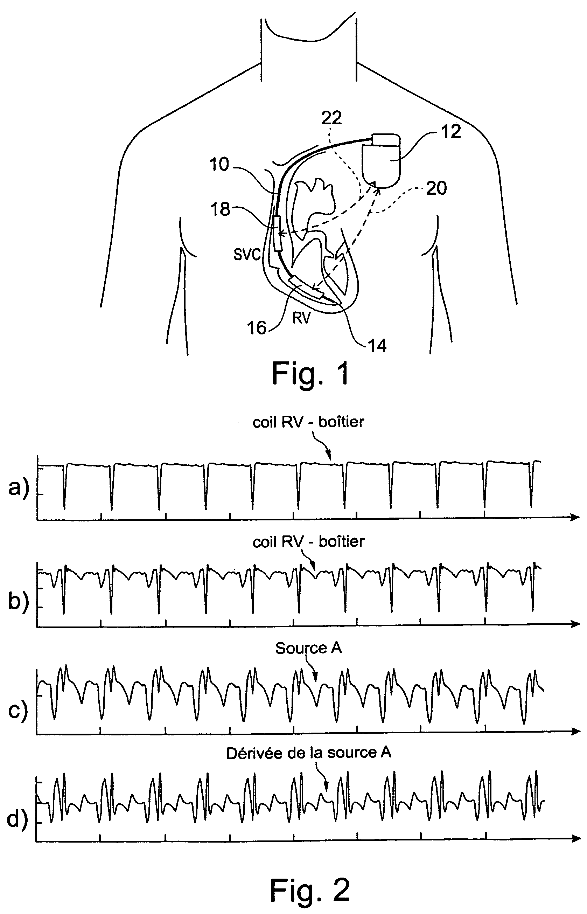

Sur la Figure 1, la référence 10 désigne de façon générale une sonde monocorps dont la partie distale est destinée à être introduite par le réseau veineux dans les deux cavités auriculaire et ventriculaire, de manière à y détecter l'activité cardiaque et appliquer en tant que de besoin un choc de défibrillation. Cette sonde est munie à son extrémité proximale de divers éléments de connexion à un générateur 12 approprié, par exemple un défibrillateur/cardioverteur implantable de type *Paradym* et *Ovatio* de ELA Médical (Sorin Group).

La sonde 10 porte une électrode distale 14 qui est une électrode de détection/stimulation destinée à être positionnée au fond de la cavité ventriculaire droite. Elle porte également sur sa partie distale une première électrode de choc 16 en forme de bobinage, ci-après "coil RV", destinée à venir se placer dans le ventricule droit et constituant par exemple la borne négative d'application du potentiel de défibrillation. La sonde 10 porte également une deuxième électrode de choc 18 en forme de bobinage, ci-après "coil SVC", destinée à venir se placer dans la veine cave supérieure pour l'application d'un choc à l'oreillette.

Une telle sonde est d'un type standard, par exemple le modèle *Isoline* fabriqué par ELA Médical (Sorin Group), dont la structure détaillée est décrite notamment dans le EP 1 557 194 A1 (ELA Medical).

Le but de l'invention est d'utiliser une telle sonde standard pour obtenir un signal représentatif de l'activité auriculaire (ondes P), notamment pour permettre une discrimination efficace des tachyarythmies selon leur origine, auriculaire ou ventriculaire.

Le coil SVC 18, situé à proximité de l'oreillette droite, permet de recueillir l'activité auriculaire, par détection monopolaire entre cette électrode de coil SVC 18 et le boîtier métallique du générateur 12.

Toutefois, sur le signal recueilli le signal ventriculaire est également présent, souvent avec une amplitude beaucoup plus importante.

L'idée de base de l'invention consiste, pour éliminer au maximum cette contribution ventriculaire, à opérer une analyse en composantes indépendantes entre le signal monopolaire coil RV/boîtier (schématisé en 20) et le signal monopolaire coil SVC/boîtier (schématisé en 22).

L'analyse en composantes indépendantes (ACI ou ICA) est une méthode d'analyse des données qui a été proposée pour opérer la séparation aveugle de sources statistiquement indépendantes. L'objet de cette analyse est d'identifier le modèle génératif linéaire :

$$x = As,$$

où x est le vecteur des signaux observés, *s* le vecteur des signaux source *et A* la matrice de mélange des sources.

L'analyse ACI repose sur l'hypothèse que les composantes $s_i$ du vecteur $s = (s_1, s_2 ... s_n)$ sont mutuellement indépendantes, hypothèse qui en l'espèce est vérifiée car l'activité auriculaire est bien indépendante de l'activité ventriculaire (les sources sont en effet indépendantes, même si les signaux recueillis ne le sont pas).

Le vecteur x sera le vecteur des signaux observés coil SVC/boîtier et coil RV/boîtier.

On notera que ces deux signaux sont de même nature (signaux tous deux monopolaires), utilisent la même référence de potentiel (la masse du boîtier) et présentent des amplitudes comparables (du fait de la taille sensiblement la même des électrodes), ce qui facilitera la séparation des sources car la morphologie des différentes ondes restera semblable.

Ces deux signaux observés sont illustrés sur les chronogrammes a) et *b)* de la Figure 2.

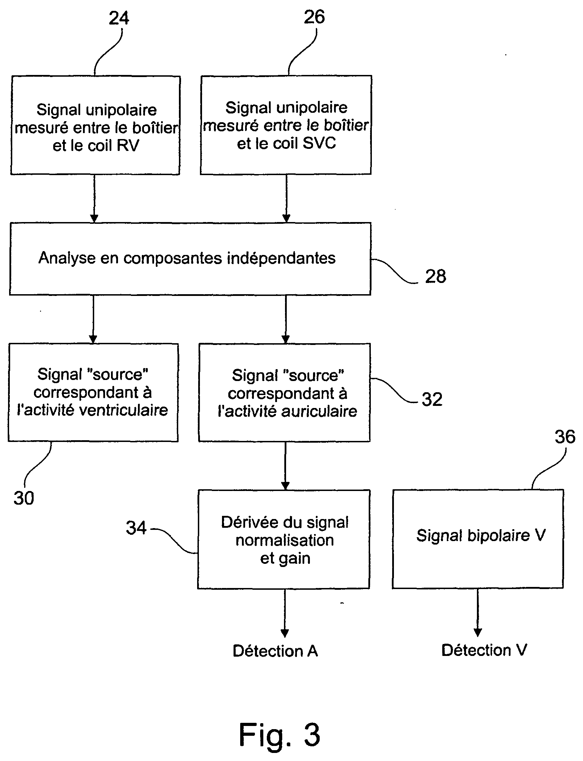

Si l'on se réfère à la Figure 3, les deux signaux coil SVC/boîtier et coil RV/boîtier captés séparément par le dispositif (blocs 24 et 26) sont soumis à l'analyse en composantes indépendantes (bloc 28). Cette analyse délivre deux composantes séparées, à savoir un signal "source" correspondant à l'activité ventriculaire (bloc 30) et un signal "source" correspondant à l'activité auriculaire (bloc 32).

L'analyse ACI est une technique en elle-même connue, qui est par exemple exposée dans l'ouvrage de Aapo Hyvärinen, Juha Karhunen et Erkki Oja, Independent Component Analysis, John Wiley & Sons, 2001, New York, auquel on pourra se référer pour de plus amples détails sur sa mise en oeuvre.

À ce stade, seul le signal source auriculaire présente un intérêt pour la suite, dans la mesure où il est possible de recueillir directement un signal ventriculaire de bonne qualité (bloc 36) au moyen de l'électrode distale 14 implantée au fond du ventricule droit.

Le signal source correspondant à l'activité auriculaire, issu de l'analyse ACI, est illustré sur le chronogramme c) de la Figure 2.

Une fois le signal source auriculaire identifié, celui-ci est soumis à une opération de dérivation (détermination du vecteur des différences entre deux points d'échantillonnage successifs), afin de rendre les ondes P plus pointues, et donc plus semblables aux véritables ondes P d'un signal bipolaire.

Le résultat après dérivation est illustré sur le chronogramme d) de la Figure 2. Cette opération de dérivation permet en outre au signal source auriculaire de ne pas être affaibli ni dégradé par les différents filtres et traitements préalables appliqués aux signaux recueillis par un défibrillateur conventionnel.

Dans la mesure où il existe une indétermination sur l'amplitude des sources obtenues par l'analyse ACI, le signal dérivé est ensuite normalisé, afin que sa valeur absolue maximale soit rendue égale à l'unité.

Le dispositif applique enfin un gain adéquat au signal ainsi dérivé et normalisé, pour que la détection des ondes P se fasse avec une sensibilité similaire à celle utilisée avec les dispositifs double chambre classiques, par exemple une valeur de gain de l'ordre de 20.

La méthode de l'invention a été testée chez quinze patients sur des signaux d'une vingtaine de secondes, avec de très bons résultats montrant une nette amélioration de la détection des ondes P par rapport à une technique conventionnelle de détection auriculaire sans traitement, à partir du signal coil SVC/boîtier.

Le signal source auriculaire obtenu et traité de la manière indiquée plus haut peut notamment être utilisé, en combinaison avec le signal de détection ventriculaire recueilli par l'électrode distale 14, pour opérer une analyse rythmologique destinée à permettre une classification des tachyarythmies.

Cette analyse est par exemple effectuée au moyen des algorithmes *PARAD, PARAD+* ou *STABILITY+* décrits dans les EP 0 626 182 A1, EP 0 838 235 A1, EP 0 813 888 A1 et EP 1 208 873 A1 (tous au nom de ELA Médical) et mis en oeuvre dans les dispositifs ELA Médical (Sorin Group).

Le signal source auriculaire obtenu et traité de la manière indiquée plus haut peut également être utilisé comme simple outil de diagnostic pour le médecin, par exemple en affichant ce signal sur l'écran d'un programmateur externe.

Si les calculs requis par l'analyse ACI sont trop complexes pour qu'ils puissent être effectués en temps réel par le microcontrôleur du générateur implanté 12, il est possible de calculer les coefficients de la matrice de mélange lors d'une phase préalable d'apprentissage, par exemple lors de séances de suivi du patient, et de mémoriser ces coefficients dans le dispositif implanté pour pouvoir les appliquer ensuite en temps réel.

Il est également possible de calculer ces coefficients pour plusieurs matrices de mélange correspondant à des situations différentes, par exemple deux positions différentes du patient (couché/debout) correspondant elles-mêmes à des positions différentes des coils SVC et RV dans le myocarde, conduisant à des morphologies de signaux sensiblement différentes.

## Revendications

**1.** Un dispositif médical implantable actif de type défibrillateur/cardioverteur implantable simple chambre, comprenant :

- une sonde monocorps (10), avec un corps de sonde portant :

une électrode de défibrillation en forme de bobinage ventriculaire (16) disposé sur le corps de manière à être positionné dans le ventricule une fois la sonde implantée, formant première électrode de choc et première électrode de détection de signaux de dépolarisation ;
une électrode de défibrillation en forme de bobinage supraventriculaire (18) disposé sur le corps de manière à être positionné dans la veine cave supérieure une fois la sonde implantée, formant deuxième électrode de choc et deuxième électrode de détection de signaux de dépolarisation ; et
une électrode distale (14) formant troisième électrode de détection de signaux de dépolarisation,

- un générateur implantable (12), comportant :

un boîtier métallique formant électrode de potentiel de référence ;
des circuits d'analyse des signaux recueillis par lesdites électrodes de détection, comprenant :

- des moyens (24) pour recueillir un premier signal unipolaire (20) entre le bobinage ventriculaire et le boîtier du générateur,
- des moyens (26) pour recueillir un deuxième signal unipolaire (22) entre le bobinage supraventriculaire et le boîtier du générateur ; et
- des moyens (28) d'analyse ACI aptes à discriminer des signaux de sources statistiquement indépendantes par Analyse en Composantes Indépendantes ; et

des circuits de délivrance de thérapies antitachycardiques,

dispositif **caractérisé en ce que** :

- la sonde est dépourvue au niveau de l'oreillette d'électrode spécifique de recueil de signal auriculaire, et
- le générateur comprend des moyens estimateurs de signal d'activité auriculaire, incluant lesdits moyens (28) d'analyse ACI,

ces moyens (28) d'analyse ACI étant aptes à opérer une analyse ACI entre lesdits premier et deuxième signaux unipolaires et à délivrer en sortie un premier signal de source représentatif de ladite activité auriculaire, séparé par l'analyse ACI d'un second signal de source représentatif d'une activité ventriculaire.

2. Le dispositif de la revendication 1, comprenant en outre :

- des moyens (34) pour transformer par dérivation ledit signal estimé après détermination par les moyens d'analyse ACI.

3. Le dispositif de la revendication 1 ou de la revendication 2, comprenant en outre :

- des moyens (34) pour normaliser ledit signal estimé à une valeur absolue maximale prédéterminée, après détermination par les moyens d'analyse ACI, le cas échéant après dérivation.

4. Le dispositif de la revendication 3, comprenant en outre :

- des moyens (34) pour appliquer un gain prédéterminé audit signal estimé après normalisation de celui-ci.

5. Le dispositif de la revendication 1, dans lequel les moyens d'analyse ACI comprennent des moyens aptes à opérer à partir d'une matrice de mélange donnée, et lesdits circuits d'analyse du générateur comprennent des moyens pour mémoriser les coefficients de cette matrice, une fois ceux-ci préalablement déterminés.

6. Le dispositif de la revendication 1, dans lequel les moyens d'analyse ACI comprennent des moyens aptes à opérer à partir d'une pluralité de matrices de mélange données, et des moyens pour sélectionner la matrice à appliquer en fonction d'au moins un critère d'état du patient déterminé par lesdits circuits d'analyse du générateur.

7. Le dispositif de la revendication 6, dans lequel ledit critère d'état est un critère de position du patient, déterminé par un capteur couplé auxdits circuits d'analyse du générateur.

8. Le dispositif de la revendication 1, comprenant en outre :

- des moyens d'analyse rythmologique, aptes à opérer une classification des tachyarythmies à partir dudit signal estimé d'activité auriculaire et d'un signal d'activité ventriculaire recueilli par la sonde du dispositif.

9. Le dispositif de la revendication 1, comprenant en outre :

- des moyens pour transmettre ledit signal estimé d'activité auriculaire à un programmateur externe, de manière à permettre l'affichage de ce signal sur un écran du programmateur à destination d'un médecin pour diagnostic.

**Patentansprüche**

1. Aktive implantierbare medizinische Vorrichtung vom Typ des implantierbaren Einkammer-Defibrillators/Kardioverters, umfassend:

- eine Monokörper-Sonde (10) mit einem Sondenkörper, der Folgendes trägt:

• eine Defibrillationselektrode in Form einer ventrikulären Wicklung (16), die auf dem Körper derart angeordnet ist, um in dem Ventrikel positioniert zu werden, nachdem die Sonde implantiert ist, die die erste Schockelektrode und die erste Elektrode zum Detektieren von Depolarisationssignalen bildet;
• eine Defibrillationselektrode in Form einer supraventrikulären Wicklung (18), die auf dem Körper derart angeordnet ist, um in der Vena cava superior positioniert zu werden, nachdem die Sonde implantiert ist, die die zweite Schockelektrode und die zweite Elektrode zum Detektieren von Depolarisationssignalen bildet; und
• eine distale Elektrode (14), die die dritte Elektrode zum Detektieren von Depolarisationssignalen bildet,

- einen implantierbaren Generator (12), umfassend:

• ein Metallgehäuse, das die Bezugspotentialelektrode bildet;
• Schaltungen zum Analysieren der Signale, die von den Elektroden zum Detektieren erfasst werden, umfassend:

- Mittel (24) zum Erfassen eines ersten unipolaren Signals (20) zwischen der ventrikulären Wicklung und dem Gehäuse des Generators,
- Mittel (26) zum Erfassen eines zweiten unipolaren Signals (22) zwischen der supraventrikulären Wicklung und dem Gehäuse des Generators; und
- Mittel (28) zur Independent Component Analysis (ICA), die geeignet sind, statistisch unabhängige Quellensignale durch Unabhängigkeitsanalyse zu unterscheiden; und

• Schaltungen zum Erteilen von antitachykarden Therapien,

wobei die Vorrichtung, **dadurch gekennzeichnet ist, dass**:

- die Sonde am Vorhof keine spezifische Elektrode zum Erfassen eines atrialen Signals aufweist, und
- der Generator Mittel zum Schätzen eines Signals der atrialen Aktivität, die die Mittel (28) zur Independent Component Analysis (ICA) einschließen, umfasst,
wobei diese Mittel (28) zur Independent Component Analysis (ICA) geeignet sind, eine Unabhängigkeitsanalyse zwischen dem ersten und dem zweiten unipolaren Signal durchzuführen und am Ausgang ein erstes Quellensignal auszugeben, das für die atriale Aktivität repräsentativ ist, das durch die Unabhängigkeitsanalyse von einem zweiten Quellensignals, das für eine ventrikuläre Aktivität repräsentativ ist, getrennt ist.

2. Vorrichtung nach Anspruch 1, ferner umfassend:

- Mittel (34), um durch Ableiten das geschätzte Signal nach dem Bestimmen durch die Mittel zur Independent Component Analysis (ICA) umzuwandeln.

3. Vorrichtung nach Anspruch 1 oder nach Anspruch 2, ferner umfassend:

- Mittel (34), um das geschätzte Signal auf einen vorbestimmten maximalen Absolutwert nach dem Bestimmen durch die Mittel zur Independent Component Analysis (ICA), gegebenenfalls nach dem Ableiten, zu normalisieren.

4. Vorrichtung nach Anspruch 3, ferner umfassend:

- Mittel (34), um eine vorbestimmte Verstärkung auf das geschätzte Signal nach dem Normalisieren von diesem anzuwenden.

5. Vorrichtung nach Anspruch 1, wobei die Mittel zur Independent Component Analysis (ICA) Mittel umfassen, die geeignet sind, ausgehend von einer gegebenen Mischmatrix zu wirken, und wobei die Schaltungen zum Analysieren des Generators Mittel umfassen, um die Koeffizienten dieser Matrix zu speichern, nachdem diese zuvor bestimmt werden.

6. Vorrichtung nach Anspruch 1, wobei die Mittel zur Independent Component Analysis (ICA) Mittel umfassen, die geeignet sind, ausgehend von mehreren gegebenen Mischmatrizen zu wirken, und Mittel, um die anzuwendende Matrix in Abhängigkeit von mindestens einem Zustandskriterium des Patienten, das durch die Schaltungen zum Analysieren des Generators bestimmt wird, auszuwählen.

7. Vorrichtung nach Anspruch 6, wobei das Zustandskriterium ein Positionskriterium des Patienten ist, das durch einen Sensor bestimmt wird, der mit den Schaltungen zum Analysieren des Generators gekoppelt ist.

8. Vorrichtung nach Anspruch 1, ferner umfassend:

- Mittel zur rhythmologischen Analyse, die geeignet sind, eine Klassifizierung der Tachyarrhythmien ausgehend von dem geschätzten Signal der atrialen Aktivität und von einem Signal der ventrikulären Aktivität, das von der Sonde der Vorrichtung erfasst wird, durchzuführen.

9. Vorrichtung nach Anspruch 1, ferner umfassend:

- Mittel, um das geschätzte Signal der atrialen Aktivität an ein externes Programmiergerät zu übertragen, um das Anzeigen dieses Signals auf einem Bildschirm des Programmiergerätes, der für einen Arzt für die Diagnose bestimmt ist, zu ermöglichen.

**Claims**

1. An active implantable medical device of the single-chamber implantable defibrillator/cardioverter type, comprising:

- a single-body lead (10), with a lead body carrying:

• a defibrillation electrode in the form of a ventricular coil (16) arranged on the body so as to be positioned in the ventricle once the lead implanted, forming a first shock electrode and a first electrode for depolarization signal detection;
• a defibrillation electrode in the form of a supraventricular coil (18) arranged on the body so as to be positioned in the superior vena cava once the lead implanted, forming a second shock electrode and a second electrode for depolarization signal detection; and
• a distal electrode (14) forming a third electrode for depolarization signal detection,

- an implantable generator (12), including:

• a metal case forming a reference potential electrode;
• circuits for analysing the signals collected by said detection electrodes, comprising:

- means (24) for collecting a first unipolar signal (20) between the ventricular coil and the generator case,
- means (26) for collecting a second unipolar signal (22) between the supraventricular coil and the generator case; and
- ICA analysis means (28) adapted to discriminate statistically independent source signals by an Independent Component Analysis; and

• circuits for delivering anti-tachycardia therapies,

the device being **characterized in that**:

- the lead is devoid, at the atrium, of a specific electrode for atrial signal collection, and
- the generator comprises atrial activity signal estimator means, including said ICA analysis means (28),
these ICA analysis means (28) being adapted to operate an ICA analysis between said first and second unipolar signals and to output a first source signal representative or said atrial activity, separated by the ICA analysis from a second source signal representative of a ventricular activity.

2. The device of claim 1, further comprising:

- means (34) for transforming said estimated signal by derivation after determination by the IAC analysis means.

3. The device of claim 1 or claim 2, further comprising:

- means (34) for normalizing said estimated signal to a predetermined maximum absolute value, after determination by the IAC analysis means, as the case may be after derivation.

4. The device of claim 1, further comprising:

- means (34) for applying a predetermined gain to said estimated signal after normalization of the latter.

5. The device of claim 1, wherein the IAC analysis means comprise means adapted to operate from a given mixing matrix, and said analysis circuits of the generator comprise means for memorizing the coefficients of this matrix, once these latter previously determined.

6. The device of claim 1, wherein the IAC analysis means comprise means adapted to operate from a plurality of given mixing matrices, and means for selecting the matrix to be applied as a function of at least one patient state criterion determined by said analysis circuits of the generator.

7. The device of claim 6, wherein said state criterion is a criterion of position of the patient, determined by a sensor coupled to said analysis circuits of the generator.

8. The device of claim 1, further comprising:

- rhythm analysis means, adapted to operate a classification of the tachyarrhythmias from said estimated signal of atrial activity and from a signal of ventricular activity collected by the lead of the device.

9. The device of claim 1, further comprising:

- means for transmitting said estimated signal of atrial activity to an external programmer, so as to allow this signal to be displayed on a screen of the programmer to a doctor for diagnosis purpose.

Fig. 1

coil RV - boîtier

a)

coil RV - boîtier

b)

Source A

c)

Dérivée de la source A

d)

Fig. 2

**Fig. 3**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0773039 A1
- US 4643201 A
- US 5628779 A
- US 6321122 A
- EP 0801960 A2
- EP 1118349 A1
- US 5776072 A
- US 5885221 A
- US 20050288600 A1
- EP 1557194 A1
- EP 0626182 A1
- EP 0838235 A1
- EP 0813888 A1
- EP 1208873 A1

**Littérature non-brevet citée dans la description**

- **AAPO HYVÄRINEN ; JUHA KARHUNEN ; ERKKI OJA.** Independent Component Analysis. John Wiley & Sons, 2001